(19) Europäisches Patentamt
European Patent Office
Office européen des brevets

(11) EP 1 080 713 A2

(12) # EUROPÄISCHE PATENTANMELDUNG

(43) Veröffentlichungstag:
**07.03.2001 Patentblatt 2001/10**

(51) Int. Cl.[7]: **A61K 7/00**, A61K 7/027, A61K 7/32

(21) Anmeldenummer: **00116237.9**

(22) Anmeldetag: **07.08.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **21.08.1999 DE 19939836**

(71) Anmelder:
**Beiersdorf Aktiengesellschaft**
**20245 Hamburg (DE)**

(72) Erfinder:
- **Gers-Barlag, Heinrich, Dr.**
  **25495 Kummerfeld (DE)**
- **Müller, Anja**
  **23843 Rümpel (DE)**
- **Petsitis, Xenia**
  **65179 Hofheim (DE)**
- **Lanzendörfer, Ghita, Dr.**
  **22087 Hamburg (DE)**
- **Kovacevic, Melanie**
  **20251 Hamburg (DE)**

(54) **Wasserhaltige kosmetische oder pharmazeutische Stifte**

(57) Kosmetische oder dermatologische Stiftzubereitungen, die feindisperse Systeme vom Typ Wasser-in-Öl darstellen, enthaltend

a) eine Ölphase, die
einen Gehalt von 10 bis 70 Gew.-%, bezogen auf das Gewicht der Fettphase, an Fett- und/oder Wachskomponenten enthält, welche oberhalb einer Temperatur von 40 °C schmelzen,
b) eine Wasserphase,
c) Bornitrid, welches sowohl hydrophile als auch lipophile Eigenschaften zeigt, welches also amphiphilen Charakter besitzt und sich an der Grenzfläche Wasser/Öl anordnet und
d) höchstens 0,5 Gew,-% eines oder mehrerer Emulgatoren

und Verfahren zu ihrer Herstellung.



Printed by Xerox (UK) Business Services
2.16.7 (HRS)/3.6

**Beschreibung**

Die vorliegende Erfindung betrifft kosmetische oder pharmazeutische Stifte, welche in Form von W/O-Emulsionen vorliegen und sich durch einen erhöhten Wassergehalt auszeichnen. Insbesondere betrifft die Erfindung dekorative Lippenstifte und Lippenpflegestifte, aber auch desodorierende oder antitranspirierend wirksame Stifte sowie Stiftformulierungen, welche beispielsweise zur Verwendung gegen Akne geeignet sind. Als weitere vorteilhafte Ausführungsformen betrifft die vorliegende Erfindung Sonnenschutzstifte und sogenannte Make-up- bzw. Kosmetikstifte, wie beispielsweise Augenbrauenstifte, Kajalstifte, Lidschattenstifte, Eye-Liner-Stifte, Abdeckstifte, Puderstifte und vergleichbare Produkte.

In der Kosmetik gibt es für Stiftformulierungen vorwiegend drei Anwendungsgebiete: die Bereiche Lippenpflege und Desodorantien und den Bereich der Kosmetikstifte, welche eine spezielle Applikationsform von Make-up-Präparaten darstellen.

Zu den Lippenstiften gehören heute zwei Produktklassen: die klassischen Lippenstifte, die vorrangig der Färbung der Lippen dienen, und die meist farblosen Pflegestifte, die entweder zur Fettung der Lippen oder zum Sonnenschutz angewendet werden.

Die Haut der Lippen besitzt nur eine äußerst dünne Hornschicht. Schweißdrüsen sind auf den Lippen gar nicht, Talgdrüsen nur vereinzelt zu finden. Daher ist die Lippenhaut praktisch frei von Fett und neigt, besonders bei kaltem und trockenem Wetter, zum Austrocknen. Dabei können sich kleine Risse in der Haut bilden, und die Empfindlichkeit der Lippen gegenüber chemischen, physikalischen und mikrobiellen Einwirkungen (z.B. Nahrungsmittel, Sonnenlicht, Herpes-Simplex-Viren) steigt.

Dies zu verhindern, ist eine Aufgabe von Lippenpflegestiften. Diese Produkte enthalten meist einen hohen Anteil an Wachsen und Fettkomponenten, die nach dem Auftragen eine abdeckende Schicht über den Lippen ausbilden.

In die Pflegestiftzubereitungen können zusätzlich Wirkstoffe eingearbeitet werden, die die Lippen pflegen oder schützen, wie z. B. Vitamine, Lichtschutzmittel, abdeckende Pigmente usw.

Dekorative Lippenstifte hingegen enthalten verschiedenste Farbpigmente. Die Auswahl der Farbstoffe erfolgt in erster Linie mit dem Ziel, einen modisch aktuellen Farbton zu erzielen. Dabei muß allerdings beachtet werden, daß alle Farbstoffe und Pigmente für Lippenstifte hinsichtlich ihrer Verwendung gesetzlichen Bestimmungen unterliegen, in Deutschland beispielsweise der Verordnung über kosmetische Mittel. Lippenstifte gehören zu den am meisten verwendeten dekorativen kosmetischen Mitteln. Ihre praktische Form erlaubt während des Tages ein mehrmaliges Auftragen und Erneuern der Farbe und des Schutzfilms.

Auch dekorative Lippenstifte enthalten zu hohen Anteilen Wachse und Fettkomponenten, die nach dem Auftragen eine abdeckende Lipidschicht über den Lippen ausbilden. Diese Lipidschicht dient als auf den Lippen haftende Grundlage für die eingearbeiteten Farbstoffe, da diese aus mancherlei Gründen nicht ohne eine solche Grundlage auf die Lippen aufgetragen werden können.

Es ist auch möglich, die Eigenschaften der pflegenden und dekorativen Lippenstifte miteinander zu kombinieren, d. h. in dekorative Lippenstifte pflegende oder schützende Substanzen einzuarbeiten.

An einen guten Lippenstift werden hinsichtlich seiner Qualität hohe Anforderungen gestellt. Dies sind zum einen anwendungstechnische Eigenschaften, zum anderen aber auch gesetzliche Bestimmungen, die beispielsweise die Verwendung der Farbstoffe regeln.

Der Verbraucher bzw. die Verbraucherin erwartet, daß ein Lippenstift eine saubere, glatte und glänzende Oberfläche besitzt. Zudem dürfen sich weder Öltröpfchen noch Wachskristalle absetzen. Auch die Applikation muß problemlos möglich sein. Lippenstifte sollen sich insbesondere glatt und ohne großen Reibungswiderstand auftragen lassen. Schon bei leichtem Andruck soll der Stift einen gut haftenden Fettfilm an die Lippen abgeben. Der erzielte Film muß die Lippen gut abdecken, möglichst lange haltbar und nicht klebrig sein sowie den Lippen Glanz verleihen. Darüber hinaus sollen Lippenstifte bruchfest und temperaturbeständig sein, und die Formulierungen dürfen nicht ausölen. Für dekorative Lippenstifte ist ferner eine hohe Haftfestigkeit der Färbung auf den Lippen wünschenswert, d. h. sie sollen die Lippen stark und wenig verwischbar färben.

Auch viele Make-up-Fromulierungen, wie z. B. Augenbrauen-, Kajalstifte usw., werden in Form von Stiften - beispielsweise in der Art von Bleistiften - angeboten. Zum Nachzeichnen und Kolorieren der Augenbrauen werden meist Formulierungen verwendet, bei denen die farbgebenden Pigmente in einer Wachs-/Öl-Basis eingelagert sind, Im allgemeinen wird die fertige Farbstoff-Wachsmasse als Mine extrudiert und in Stiften aus Rotzedernholz konfektioniert. An dekorative Augenpflegestifte stellt die Verbraucherin im Prinzip die gleichen Anforderungen wie an entsprechende Lippenpflegemittel.

Allen dekorativen Kosmetika ist gemeinsam, daß sie eine mehr oder weniger große Menge an Farbstoffen oder Farbpigmenten enthalten. Die Farbstoffe können als Bestandteile echter Lösungen vorliegen oder in Form von Pigment-Dispersionen. Auch die Kombination löslicher Farbstoffe mit unlöslichen Pigmenten wird verwendet. Die Grundlage dekorativer Kosmetika kann sehr unterschiedlich sein, aber obwohl grundsätzlich alle Möglichkeiten zur Verfügung stehen, werden hauptsächlich wasserfreie, lipophile Systeme als Grundlagen gewählt.

Von einem Deodorantstift bzw. einem Antitranspirantstift hingegen wird über die Wirksamkeit hinaus insbesondere erwartet, daß er bei der Anwendung in der Achseln keinen fettigen Eindruck erzeugt.

Sollen kosmetische oder pharmazeutische Stifte bestimmte Wirkstoffe enthalten, ist denkbar, daß die übrigen Bestandteile mit den Wirkstoffen nicht kompatibel sind. Dies ist besonders häufig der Fall, wenn die Verwendung der kosmetischen Stifte als Deo-Stifte vorgesehen ist. Antitranspirierend wirksame Stifte beispielsweise enthalten in der Regel Aluminiumchlorhydrat, welches als starke Lewis-Säure gerade für viele Stiftformulierungen nicht verwendbar ist.

Technisch betrachtet, sind insbesondere zwei Arten, Stifte zu formulieren, von besonderer Bedeutung:

Als Lippenstiftgrundmassen sowie im Bereich der Make-up-Stifte werden im allgemeinen wasserfreie Fettmischungen aus festen oder halbfesten Wachsen und flüssigen Ölen verwendet. Übliche Grundstoffe des Standes der Technik für stiftförmige Zubereitungen dieser Art sind beispielsweise flüssige Öle (z. B. Paraffinöle, Ricinusöl, Isopropylmyristat), halbfeste Bestandteile (z. B. Vaseline, Lanolin), feste Bestandteile (z. B. Bienenwachs, Ceresin und Mikrokristalline Wachse bzw. Ozokerit) und hochschmelzende Wachse (z. B. Carnaubawachs, Candelillawachs). In Bereich der Lippenpflege sind allerdings auch wasserhaltige Zubereitungen bekannt, welche gelegentlich auch in Form von W/O-Emulsionen vorliegen. Notwendige Inhaltsstoffe von Emulsionsstiftzubereitungen des Standes der Technik sind Emulgatoren, wie z. B. Polyglyceryl-3-diisostearat bzw. Polyglyceryl-3-oleat (EP-600 931-B2 bzw. EP-617 952-B2) oder Lecithin (EP-522 624-A1). Solche Stiftformulierungen zeichnen sich in der Regel durch einen Wassergehalt von deutlich weniger als 10 Gew.-% aus.

Lippenstifte, Kajalstifte und Augenbrauenstifte des Standes der Technik mit einem Gehalt an Paraffinen und Bienenwachs bzw. Japanwachs sind in "*Kosmetik, Entwicklung Herstellung und Anwendung kosmetischer Mittel", Herausgeber W. Umbach, Georg Thieme Verlag, Stuttgart - New York, 1995, S. 321 ff*, beschrieben.

Als Grundmassen für desodorierende Stifte hingegen werden in der Regel Seifen-Glykol-Gele verwendet. Diese entstehen dadurch, daß niedere Glycole und Glycerin in Gegenwart von Natriumstearat klare, transparente Gele bilden, welche zusätzlich Alkohol und Wasser aufnehmen können. Solche Formulierungen sind allerdings nicht mit Aluminiumchlorhydrat verträglich.

Der Stand der Technik hat eine Reihe von Nachteilen. Dazu zählt die Tatsache, daß lipophile Stiftformulierungen üblicherweise nur sehr wenig Wasser enthalten können, da ansonsten die Stiftkonsistenz verloren geht. Da wasserlösliche Wirkstoffe in der Regel nicht gut genug fettlöslich sind, lassen sie sich nicht in nennenswertem Maß in lipophile kosmetische Grundlagen einbauen. Ein gewisser Wassergehalt in einer Stiftformulierung ist daher durchaus erwünscht, insbesondere auch, um die Kompatibilität des Stiftes mit der menschlichen Haut zu erhöhen. Weil aber Wasser mit der hydrophoben Öl/Wachs/Emulgator-Matrix nicht kompatibel ist, sind Stifte mit hohen Wasseranteilen nach dem Stand der Technik nicht machbar.

Eine Aufgabe der vorliegenden Erfindung war es, diesen Mängeln Abhilfe zu schaffen.

Eine weitere Aufgabe der vorliegenden Erfindung war es, Zubereitungen zu entwickeln, welche als Grundlage für kosmetische Desodorantien bzw. Antitranspirantien geeignet sind und die Nachteile des Standes der Technik nicht aufweisen und welche sich weiterhin durch gute Hautverträglichkeit auszeichnen.

Da es der Haut im Lippenbereich praktisch völlig an Pigmenten mangelt, ist diese gegenüber ultravioletter Strahlung extrem empfindlich. Es empfiehlt sich daher, dem Lippenbereich einen speziellen Schutz gegen UV-Strahlung in Form von entsprechenden stiftförmigen Lichtschutzzubereitungen zukommen zulassen, insbesondere bei erhöhter UV-Exposition, wie beispielsweise im Hochgebirge. Gerade in stiftförmigen Zubereitungen des Standes der Technik werden daher oft anorganischen Pigmente als UV-Absorber bzw. UV-Reflektoren zum Schutze des Lippenbereiches vor UV-Strahlen verwendet. Dabei handelt es sich insbesondere um Oxide des Titans, aber auch gelegentlich des Zinks, Eisens, Zirkoniums, Siliciums, Mangans, Aluminiums, Cers und Mischungen davon, sowie Abwandlungen.

Ein erheblicher Mangel der Formulierungen des Standes der Technik besteht allerdings dann, daß es wegen der niedrigen Wassergehalte an sich akzeptabler Emulsionsstifte praktisch unmöglich war, wasserlösliche UV-Filtersubstanzen in solche Formulierungen einzuarbeiten. Eine weitere Aufgabe der vorliegenden Erfindung war daher auch, diesem Mangel abzuhelfen.

Aus dem DBP 23 35 549 ist ein Verfahren zur Herstellung eines kosmetischen Stiftes auf der Basis einer W/O-Emulsion bekannt. Nach dieser Lehre wird aus einer Polyhydroxyverbindung und einer nichtionogenen, oberflächenaktiven Verbindung ein Gel hergestellt, dieses mit einer kosmetischen Grundlage vermischt und Wasser in die Mischung emulgiert.

Nach diesem Verfahren sind jedoch keine Stifte herzustellen, die über die gestellten universellen Anforderungen an einen kosmetischen Stift verfügen. Da dieses Verfahren darüberhinaus kein Ein-Schritt-Verfahren darstellt, zeichnet es sich durch weitere Nachteile aus.

Die DE-OS 41 28 748 beschreibt kosmetische Stifte, welche dadurch gekennzeichnet sind, daß sie Emulsionen darstellen und als wesentliche Bestandteile Bienenwachs, einen oder mehrere Ester aus einer gesättigten Carbonsäure mit 20 - 40 Kohlenstoffatomen und einem gesättigten Alkohol mit 14 - 34 Kohlenstoffatomen, Wasser sowie

gegebenenfalls weitere Lipide und/oder übliche Hufs- und Zusatzstoffe enthalten. Obwohl diese Zubereitungen zwar vorteilhafte Eigenschaften haben, bleibt die erzielte Wirkung doch weit hinter der erhofften zurück.

Den bei weitem wichtigsten Produkttyp im Bereich der Hautpflegemittel stellen Emulsionen dar. Emulsionen sind disperse Zwei- oder Mehrphasensysteme, wobei kosmetische Emulsionen aus mindestens einer Fettphase (Fette und mineralische Öle, Fettsäureester, Fettalkohole etc.) und mindestens einer Wasserphase (Wasser, Glycerin, Glykole usw.) bestehen, die mit Hilfe von Emulgatoren fein ineinander verteilt werden.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (lipophilen) Molekülteil, die räumlich voneinander getrennt sind. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Emulgatoren setzen die Grenzflächenspannung zwischen den Phasen herab, indem sie an der Grenzfläche zwischen beiden Flüssigkeiten angeordnet sind. Sie bilden an der Phasengrenze Öl/Wasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Herkömmliche Emulgatoren können entsprechend ihrem hydrophilen Molekülteil in ionische (anionische, kationische und amphotere) und nichtionische untergliedert werden:

- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
- Wichtige Vertreter der kationischen Emulgatoren sind die quaternären Ammonium-Verbindungen.
- Der hydrophile Molekülteil nichtionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.

Durch Variation der Struktur und der Größe des polaren und des unpolaren Molekülteils lassen sich Lipophilie und Hydrophilie von Emulgatoren in weiten Grenzen verändern.

Entscheidend für die Stabilität einer (herkömmlichen) Emulsion ist die richtige Auswahl der Emulgatoren. Dabei sind die Charakteristiken aller im System enthaltenen Stoffe zu berücksichtigen. Betrachtet man z. B. Hautpflegeemulsionen, so führen polare Ölkomponenten und beispielsweise UV-Filter zu Instabilitäten. Neben den Emulgatoren werden daher in der Regel noch andere Stabilisatoren verwendet, die beispielsweise die Viskosität der Emulsion erhöhen und/oder als Schutzkolloid wirken.

An sich ist die Verwendung der üblichen Emulgatoren in kosmetischen oder dermatologischen Zubereitungen unbedenklich. Dennoch können Emulgatoren, wie letztlich jede chemische Substanz, im Einzelfalle allergische oder auf Überempfindlichkeit des Anwenders beruhende Reaktionen hervorrufen.

So ist beispielsweise bekannt, daß bestimmte Lichtdermatosen durch gewisse Emulgatoren, aber auch durch verschiedene Fette und gleichzeitige Exposition von Sonnenlicht ausgelöst werden. Solche Lichtdermatosen werden auch "Mallorca-Akne" genannt. Es hat daher nicht an Versuchen gefehlt, die Menge an üblichen Emulgatoren auf ein Minimum, im Idealfall sogar vollständig zu reduzieren.

Eine Reduktion der benötigten Emulgatormenge kann z. B. erreicht werden, wenn ausgenutzt wird, daß feinstverteilte Feststoffteilchen eine zusätzlich stabilisierende Wirkung haben. Dabei kommt es zu einer Anreicherung des festen Stoffes an der Phasengrenze Öl/Wasser in Form einer Schicht, wodurch das Zusammenfließen der dispersen Phasen verhindert wird. Die Feststoffpartikel bilden an der Grenzfläche zwischen den beiden flüssigen Phasen sozusagen eine mechanische Barriere gegen die Vereinigung der Flüssigkeitströpfchen.

Eine Möglichkeit, eine Feststoffstabilisierung in einer kosmetischen oder dermatologischen Zubereitung vorzunehmen, ist nach May-Alert (*Pharmazie in unserer Zeit, 15. Jahrg. 1986, Nr. 1, 1-7*) beispielsweise, Emulgatorgemische zu verwenden, die sowohl anionische als auch kationische Tenside enthalten. Da beim Zusammengeben von Anion- und Kationtensiden unlösliche, elektroneutrale Verbindungen ausfallen, läßt sich durch gezieltes Ausfällen dieser neutralen Tenside in der Grenzfläche Öl/Wasser eine zusätzliche Feststoffstabilisierung erreichen. Diese Emulsionen haben allerdings den Nachteil, daß die Menge an üblichen Emulgatoren im Grunde nicht reduziert wird.

Emulsionen, welche durch Feststoffe (zusätzlich) stabilisiert werden, werden nach ihrem ursprünglichen Erfinder auch als Pickering-Emulsionen bezeichnet.

Pickering-Emulsionen des Standes der Technik lassen sich - ebenso wie herkömmliche Emulsionen - nicht als Stifte formulieren, da sie in flüssiger bzw. allenfalls zähflüssiger Form vorliegen. Sie werden daher im Bereich der Hautpflege eingesetzt, beispielsweise als Haut- oder Gesichtscrème, Reinigungsmilch, Schutzlotion, Nährcrème und dergleichen mehr.

Eine weitere Aufgabe der vorliegenden Erfindung war es daher, den Stand der Technik um kosmetische oder pharmazeutische Stiftformulierungen zu bereichern, welche in Form von W/O-Emulsionen vorliegen und sich

durch einen erhöhten Wassergehalt auszeichnen.

Erstaunlicherweise werden alle diese Aufgaben gelöst durch kosmetische oder dermatologische Stiftzubereitungen, die feindisperse Systeme vom Typ Wasser-in-Öl darstellen, enthaltend

1. eine Ölphase, die
einen Gehalt von 10 bis 70 Gew.-%, bezogen auf das Gewicht der Fettphase, an Fett- und/oder Wachskomponenten enthält, welche oberhalb einer Temperatur von 40 °C schmelzen,
2. eine Wasserphase,
3. Bornitrid, welches sowohl hydrophile als auch lipophile Eigenschaften zeigt, welches also amphiphilen Charakter besitzt und sich an der Grenzfläche Wasser/Öl anordnet und
4. hächstens 0,5 Gew.-% eines oder mehrerer Emulgatoren
sowie
gegebenenfalls weitere kosmetische oder pharmazeutische Hufs-, Zusatz- und/oder Wirkstoffe enthaltend.

Es ist erfindungsgemäß besonders vorteilhaft, wenn die Stiftzubereitungen deutlich weniger als 0,5 Gew.-% eines oder mehrerer Emulgatoren enthalten. Ganz besonders bevorzugt sind erfindungsgemäße Zubereitungen, welche gänzlich frei von Emulgatoren im herkömmlichen Sinn sind.

Der amphiphile Charakter der erfindungsgemäßen Bornitridpartikel zeigt sich beispielsweise darin, daß diese sowohl in Wasser als auch in Öl dispergierbar sind.

Die erfindungsgemäßen Zubereitungen stellen Abmischungen aus Ölen bzw. öllöslichen Substanzen und Wasser bzw. wasserlöslichen Komponenten dar, die durch den Zusatz der mikronisierten Feststoffpartikel stabilisiert werden und keinen Emulgator im herkömmlichen Sinn enthalten.

Die erfindungsgemäßen Zubereitungen sind in jeglicher Hinsicht überaus befriedigende Präparate, die erstaunlicherweise höchst variabel in ihrem Wasser-Fettphasen-Verhältnis sind. Es war insbesondere überraschend, daß die erfindungsgemäßen Stiftzubereitungen einen gegenüber dem Stand der Technik deutlich erhöhten Wassergehalt aufweisen können. Sie zeichnen sich durch exzellente kosmetische Eigenschaften aus und bieten sich daher für den Einsatz in vielen Bereichen der pflegenden und dekorativen Kosmetik an. Für viele Anwendungen ist es insbesondere vorteilhaft, daß die erfindungsgemäßen Zubereitungen frei von Emulgatoren im herkömmlichen Sinne sind. Die erfindungsgemäßen Zubereitungen sind ferner hervorragende Vehikel für verschiedenste Wirkstoffe.

Der Wasserphasenanteil der erfindungsgemäßen Formulierungen wird vorzugsweise aus dem Bereich von 5 bis 80 Gew.-% gewählt, besonders vorteilhaft aus dem Bereich 10 bis 70 Gew.-%, insbesondere 15 bis 60 Gew.-%, jeweils bezogen auf das Gesamtgewicht der Formulierungen.

Vorteilhafte Bornitride im Sinne der vorliegenden Erfindung sind beispielsweise die im folgenden aufgelisteten:

| **Handelsname** | **erhältlich bei:** |
|---|---|
| Boron Nitride Powder | Advanced Ceramics |
| Boron Nitride Powder | Sintec Keramik |
| Ceram Blanche | Kawasaki |
| HCST Boron Nitride | Stark |
| Très BN® | Carborundum |
| Wacker-Bornitrid BNP | Wacker-Chemie |

Vorteilhaft ist es, den mittleren Partikeldurchmesser der verwendeten Bornitridpartikel kleiner als 20 µm, besonders vorteilhaft kleiner als 15 µm zu wählen.

Stiftzubereitungen im Sinne der vorliegenden Erfindung werden ebenfalls vorteilhaft durch Bornitridpartikel stabilisiert, die oberflächlich wasserabweisend behandelt („gecoatet") sind, wobei gleichzeitig der amphiphile Charakter gebildet werden bzw. erhalten bleiben soll.

Eine vorteilhafte Beschichtung der Bornitridpartikel besteht aus Dimethylpolysiloxan (auch: Dimethicone), einem Gemisch vollmethylierter, linearer Siloxanpolymere, die endständig mit Trimethylsiloxy-Einheiten blockiert sind. Vorteilhaft sind beispielsweise die bei der Firma Carborundum unter der Handelsbezeichnung Très BN®UHP 1106 erhältlichen, mit Dimethicone behandelten Bornitridpartikel.

Vorteilhaft ist ferner eine Beschichtung der Bornitridpartikel mit Polymethylhydrogensiloxan, einem linearen Polysiloxan, welches auch als Methicone bezeichnet wird. Vorteilhafte, mit Methicone behandelte Bornitridpartikel sind beispielsweise die bei der Firma Carborundum unter der Handelsbezeichnung Très BN® UHP 1107 erhältlichen.

Die Gesamtmenge an amphiphilem Bornitrid (eine oder mehrere Verbindungen) in den fertigen kosmetischen oder dermatologischen Zubereitungen wird vorteilhaft aus dem Bereich von 0,1 bis 15,0 Gew.-%, bevorzugt 0,5 bis 10,0 Gew.-% gewählt, bezogen auf das Gesamtgewicht der Zubereitungen.

Obwohl es besonders bevorzugt ist, die erfindungsgemäßen Zubereitungen nur durch den Zusatz von amphiphilem Bornitrid zu stabilisieren, kann es ferner vorteilhaft sein, die Bornitridpartikel mit weiteren amphiphilen Pigmenten zu kombinieren, welche gegebenenfalls auch zur Stabilisierung der Pickering-Emulsionsstifte beitragen können.

Solche Pigmente sind beispielsweise mikronisierte, anorganische Pigmente, die gewählt werden aus der Gruppe der amphiphilen Metalloxide, insbesondere aus der Gruppe Titandioxid, Zinkoxid, Siliciumdioxid bzw. Silicate (z. B. Talkum, Kaolin), wobei die Metalloxide sowohl einzeln als auch im Gemisch vorliegen können. Dabei ist es im wesentlichen unerheblich, in welcher der gegebenenfalls natürlich vorkommenden Modifikationen die verwendeten amphiphilen Metalloxide vorliegen.

Vorteilhaft ist es, den mittleren Partikeldurchmesser der zur Kombination mit Bornitrid verwendeten Pigmente zwischen 1 nm und 200 nm, besonders vorteilhaft zwischen 5 nm und 100 nm zu wählen.

Vorteilhaft im Sinne der vorliegenden Erfindung ist es, Bornitrid mit unbehandelten, nahezu reinen Pigmentpartikeln zu kombinieren, insbesondere mit solchen, welche auch als Farbstoff in der Lebensmittelindustrie und/oder als Absorber von UV-Strahlung in Sonnenschutzmitteln verwendet werden können. Vorteilhaft sind beispielsweise die bei der Firma Merck erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Erfindungsgemäß vorteilhaft ist ferner die Kombination von Bornitrid mit anorganischen Pigmenten, die oberflächlich wasserabweisend behandelt („gecoatet") sind, wobei gleichzeitig ein amphiphiler Charakter dieser Pigmente gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Ein solches Verfahren, das im folgenden am Beispiel von Titandioxid beschrieben wird, besteht beispielsweise darin, daß die hydrophobe Oberflächenschicht nach einer Reaktion gemäß

$$n\,TiO_2 + m\,(RO)_3\,Si\text{-}R' \rightarrow TiO_2\ (\text{oberfl.})$$

erzeugt wird. n und m sind dabei nach Belieben einzusetzende stöchiometrische Parameter, R und R' die gewünschten organischen Reste. Besonders vorteilhafte Kombinationspartner sind $TiO_2$-Pigmente, beispielsweise die mit Aluminiumstearat beschichteten, unter der Handelsbezeichnung MT 100 T bei der Firma TAYCA erhältlichen.

Eine weitere vorteilhafte Beschichtung der Kombinationspartner besteht aus Dimethylpolysiloxan (auch: Dimethicone), einem Gemisch vollmethylierter, linearer Siloxanpolymere, die endständig mit Trimethylsiloxy-Einheiten blockiert sind. Besonders vorteilhaft im Sinne der vorliegenden Erfindung ist die Kombination von Bornitrid mit Zinkoxid-Pigmenten, die auf diese Weise beschichtet werden.

Vorteilhaft ist ferner, wenn die neben Bornitrid verwendeten anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel beschichtet sind, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhafte Kombinationspartner sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaft im Sinne der vorliegenden Erfindung ist ferner die Kombination von Bornitrid mit einer Mischung verschiedener anorganischer, amphiphiler Pigmenttypen sowohl innerhalb eines Kristalls, beispielsweise als Eisenmischoxid oder Talkum (Magnesiumsilicat), als auch durch Mischung mehrerer Metalloxidtypen innerhalb einer Zubereitung. Besonders vorteilhafte Kombinationspartner sind Magnesiumsilicate, beispielsweise die unter der Handelsbezeichnung Talkum Micron bei der Firma Grolmann erhältlichen.

Erfindungsgemäß kann Bornitrid ferner vorteilhaft mit weiteren Pigmenten kombiniert werden, beispielsweise mit Titandioxidpigmenten, die mit Octylsilanol beschichtet sind, und/oder mit Siliciumdioxidpartikeln, die oberflächlich wasserabweisend behandelt sind. Zur Kombination geeignete Siliciumdioxidpartikel sind beispielsweise sphärische Polyalkylsilsesquioxan-Partikel wie sie in der Europäischen Offenlegungsschrift 0 686 391 erwähnt werden. Solche Polyalkylsilsesquioxan-Partikel sind beispielsweise unter den Handelsbezeichnungen Aerosil R972 und Aerosil 200V bei der Firma Degussa erhältlich.

Weiter vorteilhaft wird Bornitrid mit modifizierten Schichtsilikaten kombiniert.

Silikate sind Salze und Ester (Kieselsäureester) der Orthokieselsäure $[Si(OH)_4]$ und deren Kondensations-

produkte. Schichtsilikate (Phyllosilikate, Blattsilikate) sind (idealerweise) Silikat-Strukturen mit zweidimensional unendlichen Schichten aus $[SiO_4]^{4-}$-Tetraedern, wobei jedes Tetraeder über 3 Brücken-Sauerstoffe mit Nachbar-Tetraedern verbunden ist.

Chemische Formeln lassen sich für Schichtsilikate nur angenähert aufstellen, da sie ein großes Ionenaustausch-Vermögen besitzen und Silizium gegen Aluminium und dieses wiederum gegen Magnesium, $Fe^{2+}$, $Fe^{3}$, Zn und dergleichen ausgetauscht werden kann. Die daraus möglicherweise resultierende negative Ladung der Schichten wird in der Regel durch Kationen, insbesondere durch $Na^+$ und $Ca^{2+}$ in Zwischenschicht-Positionen ausgeglichen. Schichtsilikate können durch reversible Einlagerung von Wasser (in der 2- bis 7-fachen Menge) und anderen Substanzen wie z. B. Alkoholen, Glykolen und dergleichen mehr aufquellen.

Vorteilhafte Kombinationspartner im Sinne der vorliegenden Erfindung sind beispielsweise solche Schichtsilikate, deren größte Ausdehnungsrichtung im unmodifizierten und ungequollenen Zustand im Mittel eine Länge von weniger als 10 µm hat. Beispielsweise können die mittleren Ausdehnungen der verwendeten modifizierten Schichtsilikatpartikel bei 1000 nm x 100 nm x 1 nm und darunter liegen. Die effektive Größe der modifizierten Schichtsilikatpartikel in einer kosmetischen oder dermatologischen Formulierung hängt selbstverständlich von der Menge an eingelagerten Substanzen ab.

Vorteilhafte modifizierte Schichtsilikate im Sinne der vorliegenden Erfindung sind beispielsweise modifizierte Smektite (Smectite).

Smektite sind stets sehr feinkörnige (meist < 2 mm), überwiegend als lamellenförmige, moosartige oder kugelförmige Aggregate vorkommende Dreischicht-Tonminerale (2:1-Schichtsilikate), in denen eine zentrale Schicht aus oktaedrisch koordinierten Kationen sandwichartig von 2 Schichten aus $[(Si,Al)O_4]$-Tetraedem umgeben ist. Vorteilhafte modifizierte Smektite sind z. B. modifizierte Montmorillonite. Montmorillonite werden durch die angenäherte chemische Formel $Al_2[(OH)_2/Si_4O_{10}] \cdot n\ H_2O$ bzw. $Al_2O_3 \cdot 4\ SiO_2 \cdot H_2O \cdot n\ H_2O$ beschrieben und stellen zu den dioktaedrischen Smektiten gehörende Tonmineralien dar.

Besonders vorteilhafte Kombinationspartner im Sinne der vorliegenden Erfindung sind ferner beispielsweise modifizierte Hektorite. Hektorite gehören zu den Smektiten und haben die angenäherte chemische Formel $M^+_{0,3}(Mg_{2,7}Li_{0,3})[Si_4O_{10}(OH)_2]$, worin $M^+$ meist $Na^+$ darstellt.

Vorteilhaft im Sinne der vorliegenden Erfindung sind ferner modifizierte Bentonite. Bentonite sind Tone und Gesteine, die Smektite, vor allem Montmorillonit, als Hauptminerale enthalten. Die „Roh"-Bentonite sind entweder Calcium-Bentonite (in Großbritannien als Fuller-Erden bezeichnet) oder Natrium-Bentonite (auch: Wyoming-Bentonite).

Modifizierte Schichtsilikate im Sinne der vorliegenden Erfindung sind Schichtsilikate, insbesondere die bereits genannten Schichtsilikattypen, deren Organophilie (auch: Lipophilie) - beispielsweise durch Umsetzung mit quarternären Ammonium-Verbindungen - erhöht wurde. Solche Schichtsilikate werden auch als organophile Schichtsilikate bezeichnet.

Besonders vorteilhaft im Sinne der vorliegenden Erfindung ist es, Bornitrid mit sogenannte Bentonen zu kombinieren, d. h. mit organische Derivaten von Montmorilloniten (bzw. Bentoniten) und/oder Hectoriten, die durch Ionenaustausch-Reaktionen mit Alkylammonium-Basen hergestellt werden.

Vorteilhafte modifizierte Schichtsilikate im Sinne der vorliegenden Erfindung sind beispielsweise durch Umsetzung von Schichtsilikaten mit Quaternium-18 erhältlich. Quaternium-18 ist eine Mischung von quaternären Ammoniumchloridsalzen, welche durch die folgende Strukturformel beschrieben werden:

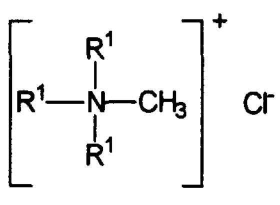

worin die Reste $R^1$ unabhängig voneinander gewählt werden aus der Gruppe Methyl und hydrierte Talgreste mit einer Kettenlänge von 12 bis 20 Kohlenstoffatomen.

Erfindungsgemäß besonders bevorzugte Kombinationspartner sind Stearalkoniumhektorit, ein Reaktionsprodukt aus Hektorit und Stearalkoniumchlorid (Benzyldimethylstearylammoniumchlorid), und Quaternium-18 Hektorit, ein Reaktionsprodukt aus Hektorit und Quaternium-18, welche z. B. unter den Handelsbezeichnungen Bentone 27 und Bentone 38 bei Nordmann & Rassmann erhältlich sind.

Weiter vorteilhaft wird Bornitrid mit mikrofeinen Polymerpartikeln kombiniert, welche in der Zubereitung in Form von Feststoffen vorliegen. Günstige Kombinationspartner im Sinne der vorliegenden Erfindung sind beispielsweise Polycarbonate, Polyether, Polyethylen Polypropylen, Polyvinylchlorid, Polystyrol, Polyamide, Polyacrylate und dergleichen mehr.

Erfindungsgemäß geeignete Kombinationspartner sind beispielsweise mikrofeine Polyamid-Partikel, insbesondere die unter der Handelsbezeichnung SP-500 bei der Firma TORAY erhältlichen. Ferner vorteilhaft sind Polyamid 6- (auch: Nylon 6) bzw. Polyamid 12- (auch: Nylon 12) Partikel. Polyamid 6 ist das aus ε-Aminocapronsäure (6-Aminohexansäure) oder ε-Caprolactam aufgebaute Polyamid [Poly(ε-caprolactam)], und Polyamid 12 ist ein Poly(ε-laurinlactam) aus ε-Laurinlactam. Vorteilhaft im Sinne der vorliegenden Erfindung sind beispielsweise Orgasol® 1002 (Polyamid 6) und Orgasol® 2002 (Polyamid 12) von der Firma ELF ATOCHEM.

Weitere vorteilhafte mikrofeine Polymerpartikel, welche sich zur Kombination mit Bornitrid eignen, sind mikrofeine Polymethacrylate. Solche Partikel sind beispielsweise unter der Handelsbezeichnung POLYTRAP® bei der Firma DOW CHEMICAL erhältlich.

Es ist insbesondere vorteilhaft, wenngleich nicht zwingend, wenn die als Kombinationspartner verwendeten mikrofeinen Polymerpartikel oberflächlich beschichtet sind. Diese Oberflächenbehandlung kann darin bestehen, daß die Polymerpartikel nach an sich bekannten Verfahren mit einer dünnen hydrophilen Schicht versehen werden. Vorteilhafte Beschichtungen bestehen beispielsweise aus Titandioxid ($TiO_2$), Zirkoniumdioxid ($ZrO_2$) oder auch weiteren Polymeren, wie beispielsweise Polymethylmethacrylat. Besonders vorteilhafte mikrofeine Polymerpartikel im Sinne der vorliegenden Erfindung sind beispielsweise die nach dem in der US-Patentschrift 4,898,913 beschriebenen Verfahren zur hydrophilen Beschichtung hydrophober Polymerpartikel erhältlichen.

Der mittlere Partikeldurchmesser der als Kombinationspartner verwendeten mikrofeinen Polymerpartikel wird vorzugsweise kleiner als 100 µm, besonders vorteilhaft kleiner als 50 µm gewählt. Dabei ist es im wesentlichen unerheblich, in welcher Form (Plättchen, Stäbchen, Kügelchen etc.) die verwendeten Polymerpartikel vorliegen.

Es ist erfindungsgemäß ferner vorteilhaft, Bornitrid mit amphiphilen modifizierten Polysacchariden zu kombinieren, welche keine verdickenden Eigenschaften zeigen.

Solche amphiphilen Polysaccharide sind beispielsweise durch Umsetzung von Stärke mit mono-, bi- oder polyfunktionellen Reagenzien bzw. Oxidations-Mitteln in weitgehend polymeranalog verlaufenden Reaktionen erhältlich.

Diese Reaktionen basieren im wesentlichen auf Umwandlungen der Hydroxy-Gruppen der Polyglucane durch Veretherung, Veresterung oder selektive Oxidation. Dabei entstehen z. B. sogenannte Stärkeether und Stärkeester der allgemeinen Strukturformel

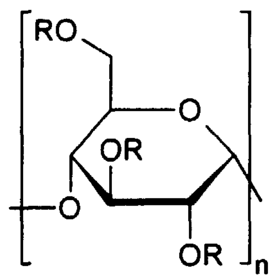

worin R beispielsweise ein Wasserstoff und/oder einen Alkyl- und/oder Aralkylrest (im Fall der Stärkeether) oder ein Wasserstoff und/oder einen organischen und/oder anorganischen Säure-Rest (im Fall der Stärkeester) darstellen kann. Stärkeether und Stärkeester sind vorteilhafte Kombinationspartner im Sinne der vorliegenden Erfindung.

Besonders vorteilhaft ist es, Bornitrid mit Stärkeethern zu kombinieren, z. B. mit solchen, die durch Veretherung von Stärke mit Tetramethylolacetylendiharnstoff erhältlich sind und welche als Amylum non mucilaginosum (nicht quellende Stärke) bezeichnet werden.

Insbesondere vorteilhaft ist ferner die Kombination von Bornitnd mit Stärkeestern und/oder deren Salzen, beispielsweise mit Natrium- und/oder Aluminiumsalzen niedrigsubstituierter Halbester der Stärke, insbesondere mit Natrium Stärke n-Octenylsuccinat der Strukturformel (I), worin R sich durch die folgende Struktur auszeichnet

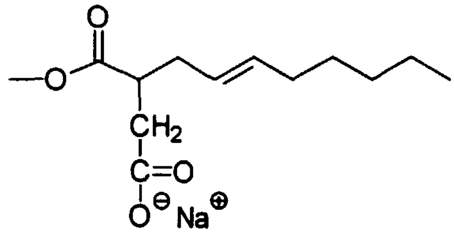

und welches z. B. unter der Handelsbezeichnung Amiogum® 23 bei der Firma CERE-STAR erhältlich ist, sowie mit Aluminium Stärke Octenylsuccinat, insbesondere mit den unter den Handelsbezeichnungen Dry Flo® Elite LL und Dry Flo® PC bei der Firma National Starch & Chemical erhältlichen.

Vorteilhaft ist es, den mittleren Partikeldurchmesser der zur Kombination mit Bornitrid verwendeten, modifizierten Polysaccharide kleiner als 20 µm, besonders vorteilhaft kleiner als 15 µm zu wählen.

Die Liste der genannten modifizierten Polysaccharide, die mit Bornitrid kombiniert werden können, soll selbstverständlich nicht limitierend sein. Modifizierte Polysaccharide, die im Sinne der vorliegenden Erfindung vorteilhafte Kombinationspartner darstellen, sind auf zahlreichen, an sich bekannten Wegen, sowohl chemischer als auch physikalischer Natur erhältlich. Zur Herstellung solcher Polysaccharide sind auch neue Wege prinzipiell denkbar. Wesentlich dabei ist, daß die modifizierten Polysaccharide amphiphile Eigenschaften zeigen und daß sie nicht verdikkend wirken.

Vorteilhaft in allen vorgenannten Fällen ist es, die Gesamtkonzentration aller Pigmente größer als 0,05 Gew.-%, besonders vorteilhaft zwischen 0,05 Gew.-% und 30 Gew.-% bezogen auf das Gesamtgewicht der Zubereitungen zu wählen, wobei die Konzentration an Bornitrid - ebenfalls bezogen auf das Gesamtgewicht der Zubereitungen - im Sinne der vorliegenden Erfindung vorzugsweise aus dem Bereich 0,05 Gew.-% bis 10 Gew.-%, vorteilhaft 0,1 Gew.-% bis 5 Gew.-% zu wählen ist.

**Wachse**

Erfindungsgemäß vorteilhaft werden die hochschmelzenden Fett- und/oder Wachskomponenten aus der Gruppe der pflanzlichen Wachse, tierischen Wachse, Mineralwachse und petrochemischen Wachse gewählt. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifizierte Wachse und synthetische Wachse, wie beispielsweise die unter den Handelsbezeichnungen Syncrowax HRC (Glyceryltribehenat), Syncrowax HGLC ($C_{16-36}$ -Fettsäuretriglycerid) und Syncrowax AW 1C ($C_{18-36}$ -Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder $C_{30-50}$ -Alkyl Bienenwachs), Polyalkylenwachse, Polyethylenglykolwachse, aber auch chemisch modifizierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise $C_{20-40}$-Alkylstearat, $C_{20-40}$-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan.

Erfindungsgemäß können die Fett- und/oder Wachskomponenten sowohl einzeln als auch im Gemisch vorliegen.

Die erfindungsgemäßen Pickering-Emulsionsstifte können vorteilhaft auch Verdickungsmittel, insbesondere auch Ölverdickungsmittel enthalten, um die taktilen Eigenschaften der Emulsion und die Stiftkonsistenz zu verbessern. Vorteilhafte Ölverdickungsmittel im Sinne der vorliegenden Erfindung sind beispielsweise weitere Feststoffe, wie z. B. hydrophobe Siliciumoxide des Typs Aerosil®, welche von der Degussa AG erhältlich sind. Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil®X R 202, Aerosil® R 805, Aerosil® R 812, Aerosil® R 972, Aerosil® R 974 und/oder Aerosil® R976.

Ferner sind auch sogenannte Metallseifen (d. h. die Salze höherer Fettsäuren mit Ausnahme der Alkalisalze) vorteilhafte Ölverdickungsmittel im Sinne der vorliegenden Erfindung, wie beispielsweise Aluminium-Stearat, Zink-Stearat und/oder Magnesium-Stearat.

Die erfindungsgemäßen Pickering-Emulsionen können als Grundlage für kosmetische oder dermatologische Stiftfomulierungen dienen. Diese können wie üblich zusammengesetzt sein und beispielsweise zur Behandlung und Pflege der Haut, als Lippenpflegeprodukt, als Deoprodukt und als Schmink- bzw. Abschminkprodukt in der dekorativen Kosmetik oder als Lichtschutzpräparat dienen. Es ist gegebenenfalls möglich und vorteilhaft, die erfindungsgemäßen Zusammensetzungen als Grundlage für pharmazeutische Formulierungen zu verwenden.

Zur Anwendung werden die erfindungsgemäßen kosmetischen und dermatologischen Zubereitungen in der für Kosmetika üblichen Weise auf die Haut in ausreichender Menge aufgebracht.

Die erfindungsgemäßen kosmetischen und dermatologischen Zubereitungen können Farbstoffe und/oder Farbpigmente enthalten, insbesondere wenn sie in Form von dekorativen Lippenstiften, Lippenkonturenstiften, Abdeckstiften, Kajalstiften, Augenkonturenstiften und/oder Augenbrauenstiften vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem *Rowe Colour Index, 3. Auflage, Society of Dyers and Colourists, Bradford, England, 1971* entnommen.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphthalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |

| Chemische oder sonstige Bezeichnung | CIN | Farbe |
|---|---|---|
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-sulfosäure)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxy-naphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8-trisulfosäure | 16290 | rot |
| 8-Amino-2 -phenylazo- 1 -naphthol-3,6-disulfosäure | 17200 | rot |
| Acid Red 1 | 18050 | rot |

| Chemische oder sonstige Bezeichnung | CIN | Farbe |
|---|---|---|
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-pyrazolon-3-carbonsäure | 19140 | gelb |
| Pigment Yellow 16 | 20040 | gelb |
| 2,6-(4'-Sulfo-2", 4"-dimethyl)-bis-phenylazo)-1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-ß-Apo-8'-Carotinaldehyd ($C_{30}$) | 40820 | orange |
| trans-Apo-8'-Carotinsäure ($C_{30}$)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(4-N-Ethyl-p-sulfobenzylamino)-phenyl-(4-hydroxy-2-sulfophenyl)-(methylen)-1-(N-ethyl-N-p-sulfobenzyl)-2,5-cyclohexadienimin] | 42053 | grün |
| Acid Blue 7 | 42080 | blau |
| (N-Ethyl-p-sulfobenzyl-amino)-phenyl-(2-sulfophenyl)-methylen-(N-ethyl-N-p-sulfo-benzyl)$\Delta^{2,5}$-cyclohexadienimin | 42090 | blau |
| Acid Green 9 | 42100 | grün |

| Chemische oder sonstige Bezeichnung | CIN | Farbe |
|---|---|---|
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethyl-N-m-sulfobenzyl-fuchsonimmonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylaminonaphthofuchsonimmonium | 44090 | grün |
| Acid Red 52 | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |

| Chemische oder sonstige Bezeichnung | CIN | Farbe |
|---|---|---|
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)-1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |

| Chemische oder sonstige Bezeichnung | CIN | Farbe |
|---|---|---|
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydroxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Manganammoniumdiphosphat | 77742 | violett |
| Manganphosphat; $Mn_3(PO_4)_2 \cdot 7\ H_20$ | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalze, Anthocyane | | rot |
| Aluminium-, Zink-, Magnesium- und Calciumstearat | | weiß |
| Bromthymolblau | | blau |
| Bromkresolgrün | | grün |
| Acid Red 195 | | rot |

Sofern die erfindungsgemäßen Stiftformulierungen in Form von Lippen(pflege-)stiften vorliegen, ist es günstig, als Farbstoff eine oder mehrer Substanzen aus der folgenden Gruppe zu wählen: 2,4-Dihydroxyazobenzol, 1-(2'-Chlor-4'-nitro-1'-phenylazo)2-hydroxynaphthalin, Ceresrot, 2-(4-Sulfo-1 -naphthylazo)-1 -naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1 -(2-Sulfo-1 - naphthylazo)-2-hydroxynaphthalin-3-carbonsäure, Aluminiumsalz der 1-(4-Sulfo-1-phenylazo)-2-naphthol-6-sulfosäure, Aluminiumsalz der 1 -(4-Sulfo- 1 -naphthylazo)-2-naphthol-3,6-disulfosäure,1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5, 7-Tetrabromfluorescein, 3',4', 5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Stiftformulierungen mit einem Gehalt an Perlglanzpigmenten. Bevorzugt sind insbesondere die im folgenden aufgelisteten Arten von Perlglanzpigmenten:

1. Natürliche Perlglanzpigmente, wie z. B.

- „Fischsilber" (Guanin/Hypoxanthin-Mischkristalle aus Fischschuppen) und
- „Perlmutt" (vermahlene Muschelschalen)

2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | **Belegung / Schichtdicke** | **Farbe** |
|---|---|---|
| Silberweiße Perlglanzpigmente | $TiO_2$: 40-60 nm | silber |
| Interferenzpigmente | $TiO_2$: 60-80 nm | gelb |
| | $TiO_2$: 80- 100 nm | rot |
| | $TiO_2$: 100-140 nm | blau |
| | $TiO_2$: 120 - 160 nm | grün |
| Farbglanzpigmente | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| Kombinationspigmente | $TiO_2$ / $Fe_2O_3$ | Goldtöne |
| | $TiO_2$ / $Cr_2O_3$ | grün |
| | $TiO_2$ / Berliner Blau | tiefblau |
| | $TiO_2$ / Carmin | rot |

Besonders bevorzugt sind z.B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit $TiO_2$ und $Fe_2O_3$ beschichtete $SiO_2$-Partikel („Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Die erfindungsgemäßen kosmetischen und dermatologischen Zubereitungen können kosmetische Hilfsstoffe enthalten, wie sie üblicherweise in solchen Zubereitungen verwendet werden, z. B. Konservierungsmittel, Baktenzide, Parfüme, Substanzen zum Verhindern des Schäumens, Pigmente, Verdickungsmittel, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Eine erstaunliche Eigenschaft der erfindungsgemäßen Zubereitungen ist, daß diese sehr gute Vehikel für kosmetische oder dermatologische Wirkstoffe in die Haut sind, wobei vorteilhafte Wirkstoffe Antioxidantien sind, welche die Haut vor oxidativer Beanspruchung schützen können.

Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden. Es ist dabei vorteilhaft, Antioxidantien als einzige Wirkstoffklasse zu verwenden, etwa dann, wenn eine kosmetische oder dermatologische Anwendung im Vordergrund steht, wie z. B. die Bekämpfung der oxidativen Beanspruchung der Haut. Es ist aber auch günstig, die erfindungsgemäßen Stiftzubereitungen mit einem Gehalt an einem oder mehreren Antioxidantien zu versehen, wenn die Zubereitungen einem anderen Zweck dienen sollen, z. B. als Desodorantien oder Sonnenschutzmittel.

Besonders vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-H ydroxyfettsäuren, Palmitinsäure, Phytinsäure, Lactoferrin), $\alpha$-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin $B_1$, das Vitamin $B_{12}$ und das Vitamin $D_1$, aber auch Bisabolol, ungesättigte Fettsäuren, namentlich die essentiellen Fettsäuren (oft auch Vitamin F genannt), insbesondere die gamma-Linolensäure, Ölsäure, Eicosapentaënsäure, Docosahexaensäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit® und Neocerit®.

Die Liste der genannten Wirkstoffe bzw. Wirkstoffkombinationen, die in den erfindungsgemäßen Pickering-Emulsionsstiften verwendet werden können, soll selbstverständlich nicht limitierend sein.

Günstig sind auch kosmetische und dermatologische Stiftzubereitungen, die in der Form eines Sonnenschutzmittels vorliegen. Vorzugsweise enthalten diese mindestens eine UV-A-Filtersubstanz und/oder mindestens eine UV-B-Filtersubstanz und/oder mindestens ein weiteres anorganisches Pigment aus der Gruppe der Oxide des Eisens, Zirkoniums, Siliciums, Mangans, Aluminiums, Cers und Mischungen davon sowie Abwandlungen, bei denen die Oxide die aktiven Agentien sind.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So können beispielsweise in pflegende Lippenstifte UV-A- bzw. UV-B-Filtersubstanzen eingearbeitet werden.

Vorteilhaft können erfindungsgemäße Zubereitungen Substanzen enthalten, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15 Gew.-% beträgt, bezogen auf das Gesamtgewicht der Zubereitungen, um kosmetische Stifte zur Verfügung zu stellen, die die Haut vor dem gesamten Bereich der ultravioletten Strahlung schützen. Sie können auch als Sonnenschutzmittel für die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure:

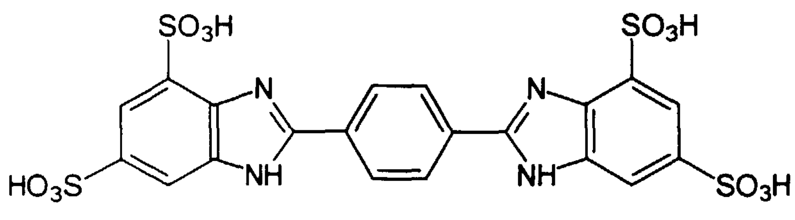

und ihre Salze, besonders die entsprechenden Natrium-, Kalium- oder Triethanolammonium-Salze, insbesondere das Phenylen-1,4-bis-(2-benzimidazyl)-3, 3'-5, 5'-tetrasulfonsäure-bis-natriumsalz:

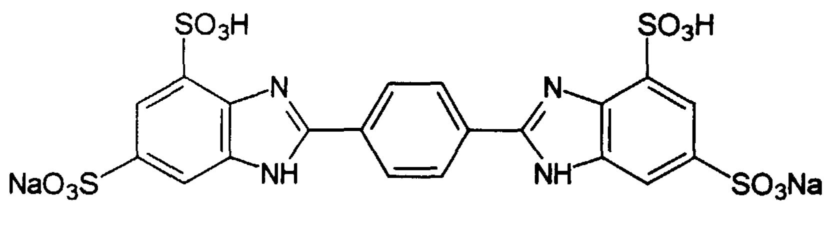

sowie das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium- oder Triethanolammonium-Salz), das auch als Benzol-,1,4-di(2-oxo-3-bornylidenmethyl-10-sulfonsäure) bezeichnet wird und sich durch die folgende Struktur auszeichnet:

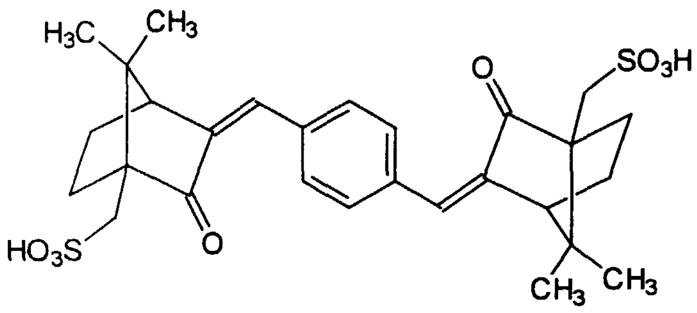

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter und/oder UV-B-Filtersubstanzen sind beispielsweise Bis-Resorcinyltriazinderivate mit der folgenden Struktur:

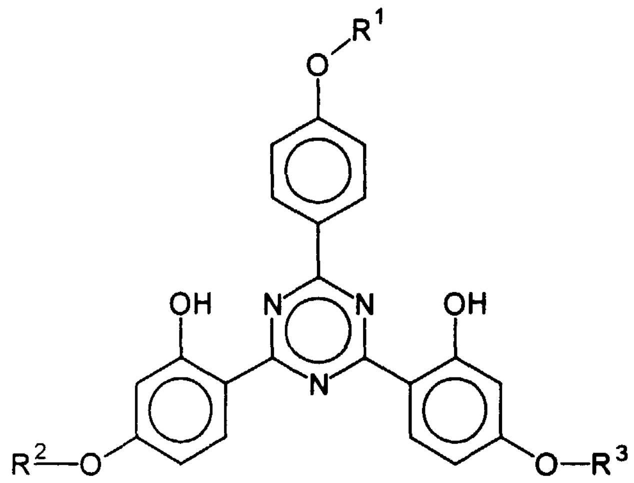

wobei $R^1$, $R^2$ und $R^3$ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3, 5-triazin (INCI: Aniso Triazin), welches unter der Handelsbezeichnung Tinosorb® S bei der CIBA-Chemikalien GmbH erhältlich ist, und das 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1 '-hexyloxy)]- 1,3,5-triazin (INCI: Octyl Triazone), welches von der BASF Aktiengesellschaft unter der Warenbezeichnung UVINUL® T 150 vertrieben wird.

Auch andere UV-Filtersubstanzen, welche das Strukturmotiv

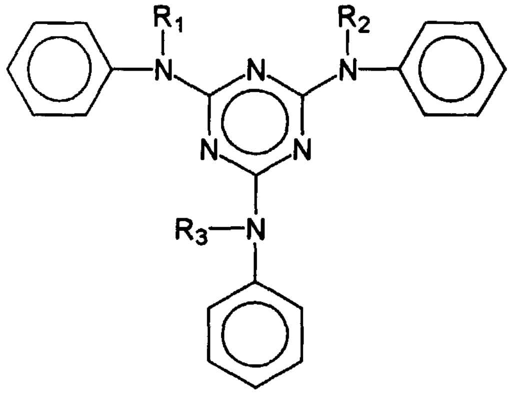

aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die genetische Formel

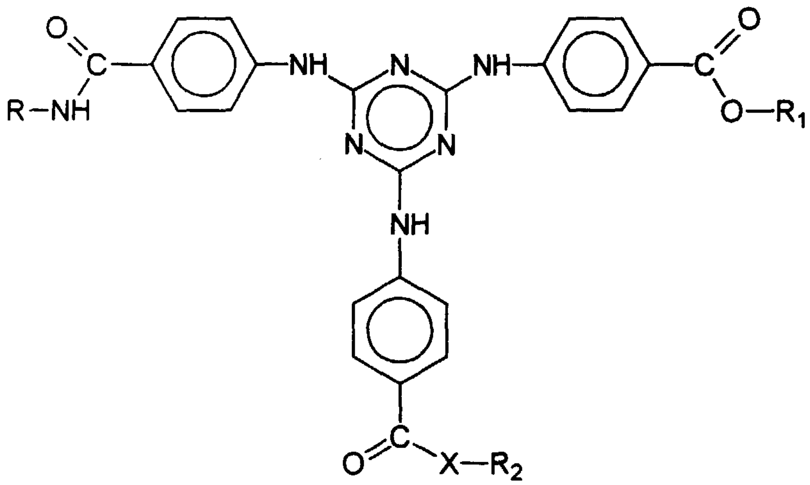

wiedergegeben wird, wobei

R einen verzweigten oder unverzweigten $C_1$-$C_{18}$-Alkylrest, einen $C_5$-$C_{12}$-Cycloalkyl-rest, gegebenenfalls substituiert mit einer oder mehreren $C_1$-$C_4$- Alkylgruppen, darstellt,

X ein Sauerstoffatom oder eine NH-Gruppe darstellt,

$R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

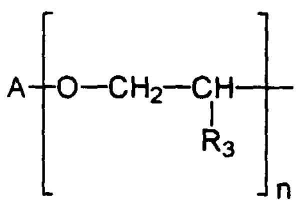

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

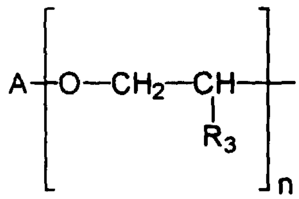

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel

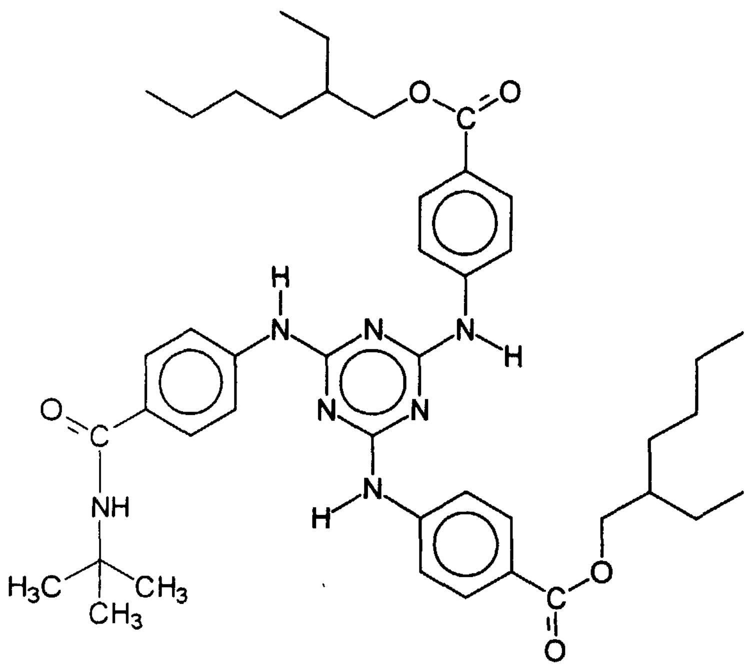

wiedergegeben wird, welches im Folgenden auch als Dioctylbutylamidotriazon (INCI: Dioctylbutamidotriazone) bezeichnet wird und unter der Handeisbezeichnung UVA-SORB HEB bei Sigma 3V erhältlich ist.

Auch in der Europäischen Offenlegungsschrift 775 698 werden bevorzugt einzusetzende Bis-Resorcinyltriazinderivate beschrieben, deren chemische Struktur durch die generische Formel

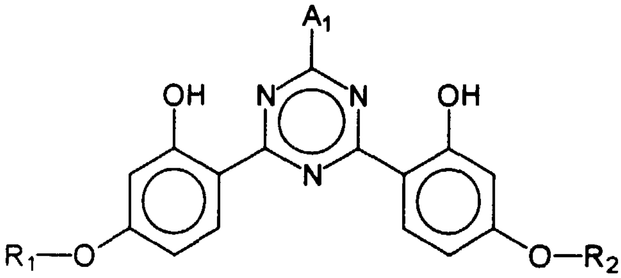

wiedergegeben wird, wobei $R_1$ , $R_2$ und $A_1$ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1, 3,5-triazin Natriumsalz, das 2,4-Bis-{[4-(3-(2-Propyloxy)-2-hydroxy-propyloxy)-2-hydroxy]-phenyl}-6-(4-methoxyphenyl)- 1,3, 5-triazin, das 2,4-Bis-{[4-(2-ethyl-hexyloxy)-2-hydroxy]-phenyl}-6-(4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]- 1,3,5-triazin, das 2,4-Bis-{[4-(2-ethyl-hexyloxy)-2-hydroxy]-phenyl}-6-(1-methyl-pyrrol-2-yl)-1,3,5-triazin, das 2 ,4-Bis-{[4-tris(trimethylsiloxy-silylpropyloxy)-2-hydroxy]-phenyl}-6-(4-methoxyphenyl)- 1,3, 5-triazin, das 2, 4-Bis-{[4-(2"-methylpropenyloxy)-2-hydroxy]-phenyl}-6-(4-methoxyphenyl)-1,3,5-triazin und das 2,4-Bis-{[4-(1',1', 1',3',5',5',5'-Heptamethylsiloxy-2"-methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel

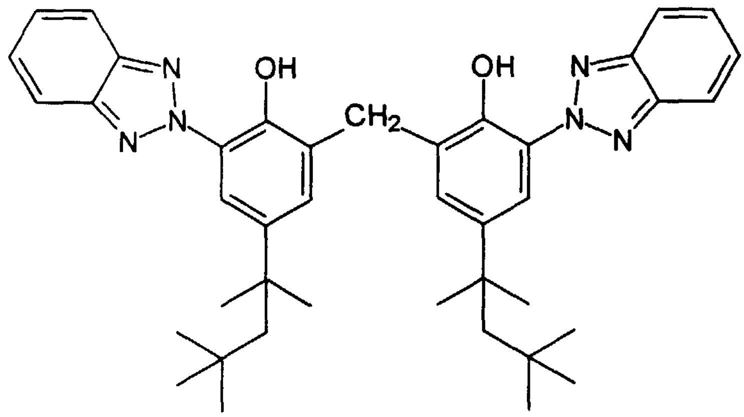

gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3, 3-tetramethyl-1 -[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel

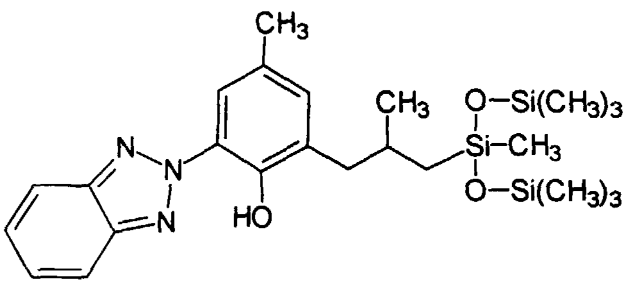

gekennzeichnet ist.

Die UV-B-Filter können öllöslich oder wasserlöslich sein. Vorteilhafte öllösliche UV-B-Filtersubstanzen sind

z. B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4, 6-Trianilino-(p-carbo-2'-ethyl-1'-hexyloxy)-1, 3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- sowie an Polymere gebundene UV-Filter.

Vorteilhafte wasserlösliche UV-B-Filtersubstanzen sind z. B.:

- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weitere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

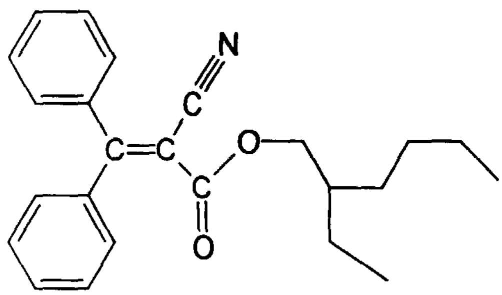

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A- und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat ( =Octylsalicylat), Homomenthylsalicylat.

Die Liste der genannten UV-Filter, die im Sinne der vorliegenden Erfindung eingesetzt werden können, soll selbstverständlich nicht limitierend sein.

Vorteilhaft können erfindungsgemäße Zubereitungen außerdem als Grundlage für kosmetische Desodorantien bzw. Antitranspirantien eingesetzt werden, so daß die vorliegende Erfindung in einer besonderen Ausführungsform Pickering-Emulsionen als Grundlage für kosmetische Deo-Stifte betrifft.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen W/O-Emulsionsstifte eingearbeitet zu werden. Vorteilhafte Substanzen sind zum Beispiel 2,4, 4'-Trichlor-2'-hdroxydiphenylether (Irgasan), 1,6-Di-(4-chlorphenylbiguanido)-hexan (Chlorhexidin), 3,4,4'-Trichlorcarbanilid, quatemäre Ammoniumverbindungen, Nelkenöl, Minzöl, Thymianöl, Triethylcitrat, Farnesol (3,7,11 -Trimethyl-2,6,10-dodecatriën-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Die Liste der genannten Wirkstoffe bzw. Wirkstoffkombinationen, die in den erfindungsgemäßen Pickering-Emulsionsstiften verwendet werden können, soll selbstverständlich nicht limitierend sein.

Die erfindungsgemäßen kosmetischen Desodorantien können in Form von aus normalen Behältern auftragbaren wasserhaltigen, kosmetischen Stiften vorliegen.

Die Menge der Antitranspiranswirkstoffe oder Desodorantien (eine oder mehrere Verbindungen) in den Zubereitungen beträgt vorzugsweise 0,01 bis 30 Gew.-%, besonders bevorzugt 0,1 bis 20 Gew.-%, insbesondere 1 bis 10 Gew.-%, bezogen auf das Gesamtgewicht der Zubereitung.

Die erfindungsgemäßen Stifte sind ferner hervorragende Vehikel für dermatologische Wirkstoffe. Insbesondere eignen sie sich als Träger für gegen Akne wirksame Substanzen. Akne ist eine Hauterkrankung mit verschiedenen Formen und Ursachen, gekennzeichnet durch nicht entzündliche und entzündliche Knötchen, ausgehend von verstopften Haarfollikeln (Komedonen), die zur Pustel-, Abszeß- und Narbenbildung führen kann. Am häufigsten ist die Acne vulgaris, die vorwiegend in der Pubertät auftritt. Ursächliche Bedingungen für die Acne vulgans sind die Verhornung und Verstopfung der Haarfollikel-Mündung, die vom Blutspiegel der männlichen Sexualhormone abhängige Talgproduktion und die Produktion freier Fettsäuren und gewebeschädigender Enzyme durch Bakterien (*Propionibacterium acnes*).

Daher ist es vorteilhaft, den erfindungsgemäßen Zubereitungen gegen Akne wirksame Substanzen zuzugeben, die beispielsweise gegen *Propionibacterium acnes* wirksam sind (etwa solche, die in DE-OS 42 29 707, DE-OS 43 05 069, DE-OS 43 07 976, DE-OS 43 37 711, DE-OS 43 29 379 beschrieben werden) aber auch andere gegen Akne wirksame Substanzen, beispielsweise all-trans-Retinsäure, 13-cis-Retinsäure und verwandte Stoffe) oder antientzündliche Wirkstoffe, beispielsweise Batylalkohol (α-Octadecylglycerylether), Selachylalkohol (α-9-Octadecenylglycerylether), Chimylalkohol (α-Hexadecylglycerylether) und/oder Bisabolol sowie Antibiotika und/oder Keratolytika.

Keratolytika sind Stoffe, die verhornte Haut (wie z. B. Warzen, Hühneraugen, Schwielen und dergleichen mehr) erweichen, damit sich diese leichter entfernen läßt oder damit sie abfällt bzw. sich auflöst.

Alle gängigen gegen Akne wirksamen Substanzen können vorteilhaft genutzt werden, insbesondere Benzoylperoxid, Bituminosulfonate (Ammonium-, Natrium- und Calcium-Salze von Schieferöl-Sulfonsäuren), Salicylsäure (2-Hydroxybenzoesäure), Miconazol (1-[2-(2,4-Dichlorbenzyloxy)-2-(2,4-dichlorphenyl)-ethyl]-imidazol) und Derivate, Adapalen(6-[3-(1-Adamantyl)-4-methoxyphenyl]-2-naphthoesäure), Azelainsäure (Nonandisäure), Mesulfen (2,7-Dimethylthianthren, $C_{14}H_{12}S_2$) sowie Aluminiumoxid, Zinkoxid und/oder feinverteilter Schwefel.

Die Menge der Antiaknemittel (eine oder mehrere Verbindungen) in den Zubereitungen beträgt vorzugsweise 0,01 bis 30 Gew.-%, besonders bevorzugt 0,1 - 20 Gew.-%, insbesondere 1 - 10 Gew.-%, bezogen auf das Gesamtgewicht der Zubereitung.

Erfindungsgemäße W/O-Pickering-Emulsionsstifte sind erhältlich, indem man zunächst die Pigmente in der Fettphase dispergiert und die Fettphase anschließend mit der Wasserphase vereinigt.

Gegenstand der Erfindung ist daher auch ein Verfahren zur Herstellung der erfindungsgemäßen Pickering-Emulsionsstifte, das dadurch gekennzeichnet ist, daß man die modifizierten Schichtsilikatpartikeln in an sich bekannter Weise in der Fettphase, welche 10 bis 70 Gew.-%, bezogen auf das Gewicht der Fettphase, an Fett- und/oder Wachskomponenten enthält, welche oberhalb einer Temperatur von 40 °C schmelzen, und gewünschtenfalls kosmetische oder pharmazeutische Hilfs-, Zusatz- und/oder Wirkstoffe enthält, bei gleichmäßigem Rühren und gegebenenfalls unter Erwärmen dispergiert und während des Homogenisiervorgangs die Wasserphase, welche gewünschtenfalls ebenfalls kosmetische oder pharmazeutische Hilfs-, Zusatz- und/oder Wirkstoffe enthält, mit der Fettphase vermischt.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**Beispiele:**

| 1. Moisturizing Lipstick | |
|---|---|
| | **Gew.-%** |
| Carnauba Wachs | 1,5 |
| Candelilla Wachs | 4 |
| Bienenwachs | 2 |
| Microcristallines Wachs | 1 |
| Jojoba Öl | 3 |
| Lanolin Öl | 3 |
| CI 77891 ($TiO_2$) | 3 |
| CI 15880:1 (D&C Red 7) | 2,3 |
| CI 73360 (D&C Red 30) | 0,7 |
| Bornitrid | 5 |
| Tocopherol | 0,1 |
| Lecithin | 0,5 |
| Glycerin | 3 |
| Wasser | 50 |
| Parfüm, Konservierungsmittel, Antioxidantien | q.s. |
| Ricinus communis | ad 100 |

| 2. Lippenstift mit Ceramid | |
|---|---|
| | **Gew.-%** |
| Stearylalkohol | 2 |
| Behenylalkohol | 2 |
| Ceramid 3 | 0,2 |
| C18-C38 Alkyl Hydroxystearoyl Stearate | 4 |
| Candelilla Wachs | 4 |
| Shea Butter | 4 |
| Lanolin Öl | 8 |
| Macadamia Öl | 8 |
| CI 15880:1 (D&C Red 7) | 2 |
| CI 17200 (D&C Red 33) | 1,5 |
| CI 42010:2 (FD&C Blue 1) | 0,5 |

(fortgesetzt)

| 2. Lippenstift mit Ceramid | |
|---|---|
| | **Gew.-%** |
| Cl 77163 (BiOCl) | 3 |
| Bornitrid | 3 |
| Tromethamine Magnesium Aluminum Silicate (Veegum Pro, Vanderbilt) | 0,1 |
| Panthenol | 5 |
| Wasser | 40 |
| Parfüm, Konservierungsmittel, Antioxidantien | q.s. |
| Ricinus communis | ad 100 |

| 3. Lippenstift mit hoher Deckkraft | |
|---|---|
| | **Gew.-%** |
| Jojoba Öl | 10 |
| Isopropylpalmitat | 5 |
| Eucerit | 0,3 |
| $C_{20}$-$C_{40}$ Alkohole (Luzatto & Figlio) | 3 |
| Carnauba Wachs | 2,8 |
| Candelilla Wachs | 4,2% |
| Bienenwachs | 2,8 |
| Mikrokristallines Wachs | 2,8 |
| Polyethylen | 2 |
| Cl 77891 ($TiO_2$) | 3 |
| Cl 45380 (D&C Red 21) | 0,8 |
| Cl 45380:3 (D&C Red 22) | 3 |
| Cl 45410:2 (D&C Red 28) | 2,5 |
| Silica and Mica (Micronasphere M, Merck) | 5 |
| Bornitrid | 3 |
| Wasser | 30 |
| Parfüm, Konservierungsmittel, Antioxidantien | q. s. |
| Ricinus communis | ad 100 |

| 4. Lippenstift mit langer Haltbarkeit | |
|---|---|
| | **Gew.-%** |
| Dimethicone Copolyol Candelilla | 7 |
| Mikrokristallines Wachs | 8 |
| Hydrogenated Polyisobutene (Polysnylan, Nippon) | 6 |
| $C_{18-38}$ Alkylhydroxystearoylstearat | 1 |
| Cyclomethicone (DC 344, Dow Corning) | 28 |
| Methicone and $TiO_2$ (methicone treated $TiO_2$, US Cosmetics) | 4 |
| Bornitrid (Belsil BNP, Wacker) | 5 |
| CI 17200 (D&C Red 33) | 1,5 |
| CI 45380:3 (D&C Red 22) | 2 |
| Octylsalicylat | 2 |
| Octylmethoxycinnamat | 2 |
| Wasser | 40 |
| Parfüm, Konservierungsmittel, Antioxidantien | q.s. |

| 5.Lipgloss | |
|---|---|
| | **Gew.-%** |
| Dimethicone Copolyol Candelilla | 8,5 |
| Mikrokristallines Wachs | 6 |
| Squalane | 6 |
| $C_{18-38}$ Alkylhydroxystearoylstearat | 1,5 |
| Lanolin Öl | 5 |
| Brassica Campestris / Aleurites fordi Öl (Glossamer L-6600, Tri-K) | 10 |
| CI 45410:2 (D&C Red 28) | 1,8 |
| CI 45370:2 (D&C Orange 5) | 0,6 |
| CI 77492 (Eisenoxid, gelb) | 2 |
| Iron Oxide with Aluminum and Mangan Oxide (Sicopearl, BASF) | 3 |
| Octylsalicylat | 2 |
| Wasser | 30 |
| Bornitrid | 2,5 |
| Parfüm, Konservierungsmittel, Antioxidantien | q.s. |
| Ricinus communis | ad 100 |

| 6. Lippenstift mineralölfrei | |
|---|---|
| | **Gew.-%** |
| Macadamia Öl | 18 |
| Octyldodecanol | 8 |
| Bienenwachs | 8 |
| Cetylpalmitate | 2 |
| Jojoba Öl | 5 |
| Carnauba Wachs | 2 |
| Tocopherolacetat | 0,75 |
| CI 77891 ($TiO_2$) | 1,8 |
| CI 45380 (D&C Red 21) | 0,8 |
| CI 45380:3 (D&C Red 22) | 2,2 |
| CI 45410:2 (D&C Red 28) | 1,6 |
| Silica and Mica (Micronasphere M, Merck) | 5 |
| Wasser | 50 |
| Bornitrid | 4,5 |
| Parfüm. Konservierungsmittel, Antioxidantien | q,s. |
| Squalan | ad 100 |

| 7. Luster Foundation Stick | |
|---|---|
| | **Gew.-%** |
| Carnauba Wachs | 1,8 |
| Bienenwachs | 1,2 |
| Ozokerite | 0,8 |
| Stearyl Methicone (Belsil SM 6018, Wacker) | 1,5 |
| Dimethicone | 8 |
| Petrolatum | 2 |
| Isostearylisostearat | 5 |
| Silica coated with $TiO_2$ and $Fe_2O_3$ (Ronasphe-. res LDP, Merck) | 5 |
| Titanium Dioxide (and) Mica (Timiron, Merck) | 2 |
| Bornitrid | 4 |
| Glycerin | 3 |
| Parfüm, Konservierungsmittel, Antioxidantien | q.s. |
| Wasser | ad 100 |

| 8. Foundation Stick matte | |
|---|---|
| | **Gew.-%** |
| Dimethicone / Vinyl Dimethicone Crosspolymer | 2 |
| Hydrolysed Beeswax | 0,5 |
| Hydrogenated Jojobaoil | 2 |
| Stearyl Methicone (Belsil SM 6018, Wacker) | 1,5 |
| Dimethicone | 8 |
| Shea Butter | 3 |
| Isostearylisostearat | 5 |
| $TiO_2$ | 1,5 |
| Kaolin | 1,2 |
| Cl 77491 (Eisenoxid rot) | 0,3 |
| Cl 77492 (Eisenoxid gelb) | 0,6 |
| Cl 77499 (Eisenoxid schwarz) | 0,15 |
| Nylon-12 (Orgasol Nummer, Elf Atochem) | 0,8 |
| Octylsalicylat | 2 |
| Octyl Triazone (Uvinul T 150) | 2 |
| t-Butyl Methoxydibenzoylmethan (Parsol 1789) | 1 |
| Glycerin | 5 |
| Bornitrid | 5 |
| EDTA | 0,5 |
| Parfüm Konservierungsmittel, Antioxidantien | q.s. |
| Wasser | ad 100 |

| 9. Sonnenschutzstift | |
|---|---|
| | **Gew.-%** |
| Octyldodecanol | 10 |
| $C_{12-15}$ Alkylbenzoat | 15 |
| Octyltriazon | 2 |
| 4-Methylbenzyliden Campher | 2 |
| Butylmethoxydibenzoylmethan | 2 |
| Vitamin E-Acetat | 0,5 |
| Konservierungsmittel | 0,5 |
| Bienenwachs | 8 |

(fortgesetzt)

| 9. Sonnenschutzstift | |
|---|---|
| | Gew.-% |
| Carnaubawachs | 2 |
| Bornitrid | 3,5 |
| Glycerin | 10 |
| Phenylbenzimidazolsulfonsäure | 2 |
| NaOH (45%ige Lösung in Wasser) | 0,7 |
| Wasser | 40 |
| Caprylic/Capric Triglycerid | ad 100 |

| 10. Mikropigmentstift | |
|---|---|
| | Gew.-% |
| Octyldodecanol | 10 |
| Dicaprylylether | 10 |
| Vitamin E-Acetat | 0,5 |
| Konservierungsmittel | 0,5 |
| Kesterwachs K82H ($C_{20-40}$ Alkylstearat) | 8 |
| Carnaubawachs | 4 |
| Vaseline | 5 |
| Bornitrid | 3 |
| Titandioxid | 5 |
| Zinkoxid | 5 |
| Glycerin | 10 |
| Wasser | 30 |
| Caprylic/Capric Triglycerid | ad 100 |

| 11. Deostift | |
|---|---|
| | Gew.-% |
| Caprylic/Capric Triglycerid | 15 |
| Octyldodecanol | 10 |
| Dicaprylylether | 5 |
| Glycerylmonolaurat | 0,5 |
| Kesterwachs K82H ($C_{20-40}$ Alkylstearat) | 15 |

(fortgesetzt)

| 11. Deostift | |
|---|---|
| | Gew.-% |
| Bornitrid | 1 |
| Glycerin | 5 |
| Aluchlorhydrat | 5 |
| Wasser | 35 |
| Ricinus communis | ad 100 |

**Patentansprüche**

1. Kosmetische oder dermatologische Stiftzubereitungen, die feindisperse Systeme vom Typ Wasser-in-Öl darstellen, enthaltend

   a) eine Ölphase, die
   einen Gehalt von 10 bis 70 Gew.-%, bezogen auf das Gewicht der Fettphase, an Fett- und/oder Wachskomponenten enthält, welche oberhalb einer Temperatur von 40 °C schmelzen,
   b) eine Wasserphase,
   c) Bornitrid, welches sowohl hydrophile als auch lipophile Eigenschaften zeigt, welches also amphiphilen Charakter besitzt und sich an der Grenzfläche Wasser/Öl anordnet und
   d) höchstens 0,5 Gew.-% eines oder mehrerer Emulgatoren.

2. Zubereitung nach Anspruch 1, dadurch gekennzeichnet, daß sie emulgatorfrei sind.

3. Zubereitung nach Anspruch 1 oder 2, dadurch gekennzeichnet, daß der Wasserphasenanteil aus dem Bereich von 15 bis 60 Gew.-% gewählt wird, bezogen auf das Gesamtgewicht der Zubereitungen.

4. Zubereitung nach einem der vorhergehenden Ansprüche, dadurch gekennzeichnet, daß weitere kosmetische oder pharmazeutische Hilfs-, Zusatz- und/oder Wirkstoffe enthalten sind.

5. Zubereitung nach einem der vorhergehenden Ansprüche, dadurch gekennzeichnet, daß der Gehalt an Bornitrid (eine oder mehrere Verbindungen) zwischen 0,1 Gew.-% und 15 Gew.-% ist, bezogen auf das Gesamtgewicht der Zubereitungen.

6. Zubereitungen nach einem der vorhergehenden Ansprüche, dadurch gekennzeichnet, daß der mittlere Partikeldurchmesser der verwendeten Bornitridpartikel kleiner als 20 μm, besonders vorteilhaft kleiner als 15 μm ist.

7. Zubereitung nach einem der vorhergehenden Ansprüche, dadurch gekennzeichnet, daß neben einem oder mehreren Bornitrid weitere Pigmente enthalten sind, insbesondere modifizierte Schichtsilikate und/oder modifizierte Polysaccharide und/oder mikrofeine Polymerpartikel und/oder mikronisierte, anorganische Pigmente, die gewählt werden aus der Gruppe der amphiphilen Metalloxide, insbesondere aus der Gruppe Titandioxid, Zinkoxid, Eisenoxide bzw. Eisenmischoxide, Siliciumdioxid bzw. Silicate, wobei die Pigmente sowohl einzeln als auch im Gemisch vorliegen können.

8. Zubereitung nach einem der vorhergehenden Ansprüche, dadurch gekennzeichnet, daß sie in Form eines Make-up- und/oder Kosmetikstiftes, insbesondere eines Augenbrauenstiftes, Kajalstiftes, Lidschattenstiftes, Eye-Liner-Stiftes, Abdeckstiftes, Puderstiftes oder dekorativen und/oder pflegenden Lippenstiftes vorliegt und zusätzlich mindestens einen Farbstoff und/oder ein Farbpigment enthält.

9. Zubereitung nach einem der vorhergehenden Ansprüche, dadurch gekennzeichnet, daß sie einen oder mehrere Zusatz- oder Wirkstoffe aus der Gruppe der Adstringentien und/oder der Antioxidantien und/oder der UV-Filtersubstanzen und/oder der antimikrobiell wirksamen Stoffe und/oder der gegen Akne wirksamen Stoffe enthält.

10. Verfahren zur Herstellung von Pickering-Emulsionsstiften, dadurch gekennzeichnet, daß modifizierte Schichtsilikatpartikel in an sich bekannter Weise in der Fettphase, welche einen Gehalt von 10 bis 70 Gew, % bezogen auf das Gewicht der Fettphase. an Fett- und/oder Wachskomponenten, welche oberhalb einer Temperatur von 40 °C schmelzen, und gewünschtenfalls kosmetische oder pharmazeutische Hilfs- Zusatz- und/oder Wirkstoffe enthält, bei gleichmäßigem Rühren und gegebenenfalls unter Erwärmen dispergiert werden und während des Homogenisiervorgangs die Wasserphase, welche gewünschtenfalls ebenfalls kosmetische oder pharmazeutische Hilfs-, Zusatz- und/oder Wirkstoffe enthält, mit der Fettphase vermischt wird.